# EUROPÄISCHE PATENTSCHRIFT

(11) **EP 0 513 783 B1**
(45) Veröffentlichungstag und Bekanntmachung des Hinweises auf die Patenterteilung: **06.12.1995**
(21) Anmeldenummer: 92108147.7
(22) Anmeldetag: 14.05.1992
(51) Int. Cl.: C07C 17/395, C07C 19/14

(54) **Verfahren zur Entfernung von Verunreinigungen aus perfluorierten Alkylbromiden oder Alkylenibromiden**
Method for the removal of contaminants from perfluorinated alkyl bromides or alkylene dibromides
Procédé pour l'enlèvement d'impuretés de bromures d'alkyles perfluorés ou de dibromures d'alkylène perfluorés

(30) Priorität: 17.05.1991 DE 4116121
(43) Veröffentlichungstag der Anmeldung: 19.11.1992
(73) Patentinhaber: HOECHST AKTIENGESELLSCHAFT, 65926 Frankfurt am Main (DE)
(72) Erfinder: Raab, Klaus, Dr., W-8269 Burgkirchen (DE)

(56) Entgegenhaltungen:
- EP-A- 0 151 697
- EP-A- 0 401 493
- DE-A- 4 116 361
- DE-B- 1 219 460
- PATENT ABSTRACTS OF JAPAN vol. 15, no. 445 (C-0884) 13. November 1991 & JP-A-03 190 826 (TOKUYAMA SODA CO LTD) 20. August 1991

## Beschreibung

Die Erfindung betrifft ein Verfahren zur Reinigung von unreinen perfluorierten Alkylbromiden oder Alkylendibromiden, die im wesentlichen als Verunreinigungen geringe Mengen von perfluorierten Alkyliodiden oder Alkylendiiodiden, im folgenden "Iodalkanverbindungen" genannt, enthalten.

Perfluorierte Alkylbromverbindungen, beispielsweise Perfluorhexylbromid oder Perfluoroctylbromid, werden unter anderem für medizinische Zwecke eingesetzt, wobei sie zum Beispiel intravenös oder gastrointestinal appliziert (in den Körper des Patienten eingeführt) werden. Sie dienen zum Beispiel als Kontrastmittel bei der Untersuchung mit Röntgenstrahlen oder mit Ultraschall, zur Erkennung von Tumoren, zur Organperfusion und in wäßriger Emulsion als Blutersatzstoff. Für diese Anwendungsgebiete werden außergewöhnlich hohe Reinheitsforderungen gestellt, um unerwünschte physiologische Wirkungen von Verunreinigungen weitgehend einzuschränken oder ganz zu vermeiden.

Perfluorierte Alkylbromverbindungen, wie sie beispielsweise für die obengenannten Zwecke verwendet werden, können auf verschiedenen Wegen hergestellt werden, die aber in der Regel Produkte ergeben, die den medizinischen Reinheitsforderungen nicht entsprechen und entweder für diese Anwendungsgebiete nicht einsatzfähig sind oder kostenaufwendig und verlustreich gereinigt werden müssen.

Als mögliche Herstellverfahren für perfluorierte Alkylbromide oder Alkylendibromide kommt die Umsetzung der entsprechenden perfluorierten Iodalkanverbindungen mit elementarem Brom oder Bromiden in Frage, die jedoch zu Produkten führt, die noch Ausgangsverbindungen enthalten, welche häufig wegen nahe beieinanderliegender Siedepunkte nur schwierig abzutrennen sind.

Haszeldine, J. Chem. Soc., 1953, Seiten 3 766 und 3 767 beschreibt die Umsetzung von Perfluoralkyliodiden mit einem 10%igen Überschuß von Brom unter Bestrahlung mit ultraviolettem Licht während 7 Tagen. So werden C₃- und C₄-Perfluoralkylbromide mit 95 bis 98 % Ausbeute sowie C₅- und C₆-Perfluoralkylbromide mit 90 bis 91 % Ausbeute erhalten. Über Art der UV-Strahlung sowie eine eventuelle weitere Reinigung ist nichts ausgesagt.

Analog berichten Huang Bingnan und Huang Weiyuan, Shanghai Inst. Org. Chem. Acad. Sinica, Huaxue Xuebao, 1984, 42 (10), Seiten 1 106 bis 1 108 (C.A. 102, 78312x) über die Bromierung von Perfluoralkyliodverbindungen mit Brom unter Anwendung von UV-Strahlung. Beispielsweise kann aus Cl(CF₂)₄I mit etwas weniger als der äquimolaren Menge Brom durch Bestrahlung während 50 Stunden Cl(CF₂)₄Br mit 93 % Ausbeute erzeugt werden. Auch hier fehlen Angaben über die Art der UV-Strahlung.

In der EP 194 781 A1 ist ein Verfahren beschrieben, mit dem unter anderem perfluorierte Alkylendibromide durch Umsetzung entsprechender Alkylendihalogenide (wobei das Halogen Cl und/oder I sein kann) mit überschüssigem Brom bei Temperaturen bis 180 °C ohne Anwendung von UV-Strahlung hergestellt werden können. Beispielsweise werden bei 150 °C aus I(CF₂)₆Cl und Brom 99 % Br(CF₂)₆Br und
1 % Ausgangsverbindung erhalten.

In der deutschen Offenlegungsschrift (DE-OS) 41 16 361 wird vorgeschlagen, Perfluoralkyliodide bei 100 bis 290 °C mit elementarem Brom ohne Anwendung von UV-Strahlung umzusetzen. Es werden bei Temperaturen von 190 °C Ausbeuten an Perfluoralkylbromiden bis 97 % und Umsätze über 99,9 % erhalten. Jedoch hat dieses Verfahren, wie auch das in EP 194 781 A1 beschriebene, den Nachteil, daß für sehr hohe Umsätze hohe Temperaturen oder sehr lange Reaktionszeiten angewendet werden müssen, die wegen der Anwendung des aggressiven Broms in druckfesten Apparaturen erhebliche Korrosionsprobleme und Sicherheitsrisiken mit sich bringen sowie die Bildung unerwünschter Nebenprodukte begünstigen.

Keine besonderen Korrosionsprobleme haben Herstellverfahren für perfluorierte Alkylbromverbindungen, wie sie in DE-OS 39 37 567 (US-PS 5 073 651) beschrieben sind und in DE-OS 40 04 783 (US-PS 5 051 535) und 40 18 913 vorgeschlagen werden. Hier werden Perfluoralkyliodide mit Bromidionen, die als Salze mit bestimmten Kationen vorliegen, ohne oder mit Anwendung eines dipolaren aprotischen Lösungsmittels umgesetzt, wobei in Gegenwart bestimmter Metallkomplexverbindungen oder eines Alkalisalzes einer Hydroxyalkansulfinsäure gearbeitet werden kann, um die Ausbeuten an Perfluoralkylbromid zu verbessern.

In allen Fällen enthalten die erzeugten perfluorierten Alkyl- beziehungsweise Alkylenbromverbindungen noch Anteile an den entsprechenden Iodverbindungen, die für die weiter oben genannten Anwendungen zu hoch sind, auch wenn durch eine anschließende Destillation der Anteil an perfluorierten Alkyl- beziehungsweise Alkylenbromverbindungen wesentlich, beispielsweise auf 99 % und darüber, gesteigert wurde.

Es bestand daher die Aufgabe, ein Verfahren zu finden, das es ermöglicht, in Gemischen, die im wesentlichen perfluorierte Alkyl- und Alkylenbromverbindungen enthalten, den geringen Anteil an den entsprechenden Iodverbindungen möglichst weitgehend zu senken, um erweiterte Anwendungsmöglichkeiten der Bromverbindungen zu erreichen.

Die Aufgabe wird gelöst durch ein Verfahren zur Entfernung von Verunreinigungen aus einer Verbindung oder Verbindungen der Formel

X(CF₂)ₙBr (I) ,

in der bedeuten
X = F, (CF₃)₂CF oder Br und
n = eine Zahl von 2 bis 16,
das dadurch gekennzeichnet ist, daß das zu reinigende, vorzugsweise flüssige Gemisch mit einer elektromagnetischen Strahlung im Wellenlängenbereich von 230 bis 500 nm bestrahlt, während oder nach der Bestrahlung mit mindestens einem der folgenden Mittel: Aktivkohle, einem feinteiligen Feststoff aus der Reihe Cu, CuI, Ag, Mg, Zn, Al, Mn, Fe, Co, Ni und einem Alkalimetallborhydrid, Chlor, Brom, Sauerstoff, wäßrige Lösungen von H₂O₂, anderen anorganischen peroxidischen Verbindungen oder Alkalimetallsalzen mit folgenden Anionen: SO₃²⁻, S₂O₃²⁻, PO₃³⁻, NO₂⁻, CO₃²⁻, HCO₃⁻, BrO₃⁻, ClO₃⁻, wäßrige oder alkoholische Lösungen von Alkalimetallhydroxiden, -iodiden, -alkoholaten oder niedrigen aliphatischen Alkoholen in Kontakt gebracht, nach Beendigung der Behandlung das Mittel abgetrennt und das Produkt, falls erforderlich, mit Wasser gewaschen und destilliert wird.

Die erfindungsgemäß zu reinigenden Gemische sollten mindestens zu 95 Gew.-%, vorzugsweise zu mindestens 99 Gew.-% und insbesondere zu mindestens 99,5 Gew.-% (alle Gewichtsprozentangaben bezogen auf das Gemisch), aus einer Verbindung oder mehreren Verbindungen der Formel (I) bestehen. Wegen seiner guten Wirkung und der günstigen Verwendbarkeit der gereinigten Gemische wird das erfindungsgemäße Verfahren bevorzugt auf Gemische angewendet, die im wesentlichen aus mindestens einer Verbindung der Formel (I) bestehen, in der X = F oder Br und n = eine Zahl von 4 bis 10 bedeuten. Insbesondere werden Gemische eingesetzt, die im wesentlichen aus mindestens einer Verbindung der Formel (I) bestehen, in denen X = F ist und n eine Zahl von 6 bis 8 bedeutet.

Die zu reinigenden Gemische können gasförmig erfindungsgemäß behandelt werden, zweckmäßig werden sie jedoch im flüssigen Zustand behandelt.

Die Temperatur, bei der die Gemische bestrahlt werden, soll 0 bis 100 °C, vorzugsweise 5 bis 80 °C, über dem Schmelzpunkt des Gemisches, jedoch nicht über 200 °C liegen, da bei höheren Temperaturen unerwünschte Nebenreaktionen auftreten können. Zweckmäßig wird man im allgemeinen auch Temperaturen unter 0 °C nicht verwenden, um eine aufwendigere Kühlung zu vermeiden. Die Temperatur kann sich während der Bestrahlung innerhalb der genannten Grenzen ändern. Die Temperatur des zu behandelnden Gemisches soll während des Inkontaktbringens mit den vorstehend genannten Mitteln unmittelbar nach der Bestrahlung ebenfalls innerhalb der obengenannten Temperaturbereiche liegen; sie kann dieselbe sein wie die Temperatur bei der Bestrahlung oder unterschiedlich davon sein.

Das erfindungsgemäße Verfahren wird bei normalem Atmosphärendruck oder dem autogenen Druck der Reaktionsmischung durchgeführt.

Das erfindungsgemäß zu reinigende Gemisch soll bis zu 1 Gew.-% perfluorierte Iodalkanverbindungen enthalten. Prinzipiell läßt sich das neue Verfahren auch für Gemische verwenden, die über 1 Gew.-% perfluorierte Iodalkanverbindungen enthalten, erfordert jedoch dann meist hohe Bestrahlungsleistungen und/oder lange Bestrahlungszeiten, um einen erwünschten niedrigen Gehalt an den Iodverbindungen zu erreichen, so daß es in der Regel zweckmäßig ist, aus dem Gemisch zunächst auf anderem Wege, beispielsweise durch Destillation, größere Mengen an unerwünschten Iodverbindungen abzutrennen und erst dann das erfindungsgemäße Verfahren anzuwenden. Vorzugsweise wird ein Gemisch nach dem neuen Verfahren behandelt, das nicht mehr als 0,1 Gew.-% perfluorierte Iodalkanverbindungen enthält.

Das zu reinigende Gemisch wird mit einer elektromagnetischen Strahlung im Wellenlängenbereich von 230 bis 500 nm bestrahlt. Gute Ergebnisse werden häufig erhalten, wenn eine Strahlung verwendet wird, die zu mindestens 50 % ihrer Gesamtintensität im Wellenlängenbereich von 280 bis 450 nm liegt und deren Intensität im Wellenlängenbereich kleiner als 260 nm unter 10 % der Gesamtintensität liegt. Sofern das Mittel, mit dem das zu reinigende Gemisch in Kontakt gebracht wird, Chlor oder Brom ist, wird für die Bestrahlung vorzugsweise ein Wellenlängenbereich von 350 bis 500 nm eingesetzt.

Geeignete Strahlungsquellen sind Gasentladungslampen in Niederdruck-, Mitteldruck- oder Hochdruckausführung mit einer Füllung, beispielsweise aus Quecksilber, Edelgasen, Wasserstoff oder Deuterium. Besonders genannt sei die Quecksilberdampf-Hochdrucklampe mit Filtern, die die elektromagnetische Strahlung unterhalb etwa 230 nm, vorzugsweise unter etwa 260 nm, Wellenlänge absorbieren oder durch Lumineszenz in längerwelliges UV-Licht umwandeln, und die Xenon-Hochdrucklampe. Ferner sind Lampen geeignet, die einen hohen Anteil sichtbares Licht im kurzwelligen Teil des Spektrums (violett und blau) abstrahlen, wie zum Beispiel Spezial-Leuchtstofflampen, Typ TL/03 oder TL/52 von Firma Osram, Berlin/München, Bundesrepublik Deutschland.

Auch Glimmentladungslampen, eine Sonderform der Gasentladungslampe, mit Edelgas- oder Edelgas- und Quecksilberdampffüllung können eingesetzt werden, ebenso wie ein Xenon-Chlor-Laser (Wellenlänge 308 nm) oder Lichtbogenlampen. Geeignet können auch leistungsfähige Halogenlampen sein, die eine verbesserte Version der Wolframdrahtglühlampen darstellen.

Als Absorptionsmittel sind bei Lampen geringerer Strahlungsleistung spezielle Gläser, beispielsweise das Glas "Heralux®" oder ein Borosilikatglas mit besonders niedriger Wärmeausdehnung (®Duran 50 der Firma Heraeus/Hanau, Bundesrepublik Deutschland) geeignet. Bei Lampen höherer Leistung empfiehlt es sich, ein flüssiges Absorptionsmittel, das gleichzeitig zur Kühlung der Lampe in einem gekühlten Kreislauf dienen kann, einzusetzen. Geeignet sind beispielsweise Methanol oder Ethylenglykole.

Der Raum, in dem die erfindungsgemäße Reaktion stattfindet, sollte zweckmäßig der Strahlungsquelle angepaßt werden, so daß eine möglichst vollständige Erfassung der Strahlung durch das Reaktionsgemisch erfolgt. Hierfür sind beispielsweise Strahlungsquellen, die in das flüssige, zu reinigende Gemisch eintauchen, besonders geeignet. Sofern die Außenwandung des Reaktionsgefäßes für die Strahlung der Tauchlampe im Wellenlängenbereich 270 bis 500 nm durchlässig ist, empfiehlt es sich, einen reflektierenden Belag beziehungsweise eine entsprechende Umhüllung der Außenwandung, beispielsweise aus Aluminiumfolie, anzubringen.

Wenn das Mittel, mit dem das zu reinigende Gemisch in Kontakt gebracht wird, ausgewählt wird aus der Gruppe Sauerstoff, Chlor, Brom, niedrige aliphatische Alkohole oder wäßrige beziehungsweise alkoholische Lösungen der genannten anorganischen Verbindungen, ausgenommen die Alkalimetalliodide, kann der Kontakt während der Bestrahlung, das heißt in der gleichen Zone in der bestrahlt wird, erfolgen, wobei der Kontakt, beispielsweise durch Rühren, verstärkt werden kann.

Bei strahlungsundurchlässigen Mitteln, wie Aktivkohle oder feinteilige Metallpulver, sind zweckmäßig Bestrahlung und Behandlung getrennt. Hier ist es vorteilhaft, das zu reinigende Gemisch durch eine Zone, in der die Bestrahlung erfolgt, und anschließend durch eine Zone, in der es mit einem der genannten Mittel in Kontakt kommt, gegebenenfalls im Kreislauf mehrfach hintereinander, zu führen. Der Kontakt mit allen genannten Mitteln kann jedoch auch erst nach Abschluß der Bestrahlung erfolgen.

Die geeigneten Mittel sind weiter oben bereits beschrieben. Sofern Lösungen verwendet werden, sollten diese möglichst 0,1 g oder mehr des gelösten Stoffes je 100 g Lösung enthalten. Als niedrige aliphatische Alkohole sind solche geeignet, die 1 bis 6 C-Atome, vorzugsweise 1 bis 4 C-Atome, im Molekül enthalten. Von diesen werden zweckmäßig 1 bis 50 g je 100 g zu reinigendes Gemisch verwendet. Von Sauerstoff, Chlor oder Brom oder den anorganischen Verbindungen, wie Alkalimetallborhydrid, anorganische peroxidische Verbindungen, beispielsweise Wasserstoffperoxid, Alkalimetallpersulfate, -perborate, -persilikate, ferner Alkalimetallhydroxide, -sulfite, -thiosulfate, -phosphite, -nitrite, -carbonate, -hydrogencarbonate, -bromate oder -chlorate sowie auch von den Alkalimetallalkoholaten werden mindestens die äquivalenten Mengen, die zur Umsetzung der in dem zu reinigenden Gemisch enthaltenen Iodatome notwendig sind, eingesetzt. Vorteilhaft verwendet man jedoch einen Überschuß der anorganischen Stoffe, der bei Gehalten des Gemisches an perfluorierten Iodalkanverbindungen in der Größenordnung von 0,1 bis 1 Gew.-% das 1,5- bis 10fache, vorzugsweise das 2- bis 5fache, bei Gehalten des Gemisches an perfluorierten Iodalkanverbindungen bis zu 0,1 Gew.-% das 5- bis 500fache, vorzugsweise das 10- bis 100fache, der zur Umsetzung des Iods äquivalenten Menge beträgt.

Von Mitteln, wie konzentrierte Alkalimetalliodidlösung, Aktivkohle und feinteilige Metalle, wie Kupfer, Silber, Magnesium, Zink, Aluminium, Mangan, Eisen, Kobalt oder Nickel, kann ein beliebiger Überschuß verwendet werden, zweckmäßig setzt man 2 bis 10 g des Mittels je 100 g zu reinigendes Gemisch ein.

Vorzugsweise werden folgende Mittel angewendet: eine wäßrige Lösung eines Alkalimetallhydroxids, -carbonats, -hydrogencarbonats oder -thiosulfats, ferner Aktivkohle, feinteiliges Magnesium oder feinteiliges Zink.

In einer weiteren bevorzugten Ausführungsform des erfindungsgemäßen Verfahrens wird Chlor oder Brom zusammen mit einer elektromagnetischen Strahlung im Wellenlängenbereich von 350 bis 500 nm, wie weiter oben bereits erwähnt, eingesetzt.

Die Bestrahlung des zu reinigenden Produktes und das Inkontaktbringen mit den beschriebenen Mitteln wird fortgesetzt, bis, beispielsweise nach kapillargaschromatographischer Bestimmung, der angestrebte geringe oder nicht mehr nachweisbare Gehalt an perfluorierten Iodalkanverbindungen im zu reinigenden Gemisch festgestellt wird. Die Bestrahlungszeiten können sehr verschieden sein. Sie hängen unter anderem ab von Menge und Art der perfluorierten Iodalkanverbindungen, der Art des zugesetzten Mittels sowie von Art und Intensität der Bestrahlung. Im allgemeinen sind Bestrahlungszeiten von 0,2 bis 20 Stunden genügend.

Nach Abschluß der Bestrahlung und, falls erforderlich, einer Nachreaktionszeit mit dem angewendeten Mittel wird dieses von dem gereinigten Produkt abgetrennt, beispielsweise durch Filtration, Dekantieren, gegebenenfalls unter Verwendung von Zentrifugalkräften, durch Trennung zweier flüssiger Phasen in einem Scheidetrichter oder anderen bekannten Trennmethoden. Wenn wäßrige oder alkoholische Mittel verwendet wurden, schließt sich nun zweckmäßig das Waschen (Verrühren, Ausschütteln) des gereinigten Produktes mit Wasser und Abtrennung der Waschflüssigkeit an. Falls erforderlich, wird anschließend das gereinigte Produkt fraktioniert destilliert, wobei zweckmäßig verminderter Druck angewendet wird, wenn der Siedepunkt der Hauptfraktion bei normalem Atmosphärendruck über 200 °C liegt.

Das, wie beschrieben, gereinigte Produkt enthält einen wesentlich verminderten Gehalt an perfluorierten Iodalkanverbindungen, der je nach Ausgangsgemisch und Behandlungsbedingungen unter 0,0005 Gew.-%, bezogen auf das zu reinigende Produkt, liegen kann.

Ein solches Produkt entspricht den außergewöhnlich hohen Reinheitsforderungen, wie sie für verschiedene Anwendungen im medizinischen Sektor gestellt werden.

Das erfindungsgemäße Verfahren benötigt keine hohen Temperaturen und/oder hohen Drücke, die apparative Schwierigkeiten verursachen, insbesondere, wenn chemisch aggressive Mittel verwendet werden.

Nachfolgende Beispiele sollen die Erfindung erläutern.

### Beispiel 1

In eine zylindrische Bestrahlungsapparatur (Innendurchmesser: 5,5 cm, Höhe: 20 cm) mit Mantelkühlung, einer Heraeus-Tauchlampe mit dem Quecksilber-Hochdruckstrahler TQ 150 und einem Tauchrohr aus Duran 50 zur Absorption der kurzwelligen UV-Strahlung (Strahlungsfluß von 300 bis 580 nm: circa 30 Watt, circa 50 % der Gesamt-Strahlungsintensität liegen im Wellenlängenbereich von 350 bis 450 nm), Intensivkühler mit CaCl₂-Trockenrohr, Innenthermometer und Magnetrührfisch werden 400 g Perfluoroctylbromid der Formel CF₃(CF₂)₇Br eingefüllt. Das Perfluoroctylbromid enthält nach kapillargaschromatographischer Bestimmung 0,017 % (m/m) Perfluorhexyliodid der Formel CF₃(CF₂)₅I, 0,011 % (m/m) Perfluorheptyliodid der Formel CF₃(CF₂)₆I und 0,018 % (m/m) Perfluoroctyliodid der Formel CF₃(CF₂)₇I. Unter Bestrahlung und Kühlung wird die Lösung ohne Inertgasüberlagerung bei 38 °C gerührt. Bereits nach etwa 3 Minuten färbt sich das Perfluoroctylbromid durch Abspaltung von elementarem Iod aus den Perfluoralkyliodiden rosa. Nach 15 Minuten, 1 Stunde und 8 Stunden werden Proben gezogen und die Proben durch Ausschütteln mit 2m-Natronlauge und destilliertem Wasser vom Iod abgetrennt und kapillargaschromatographisch untersucht. Insgesamt werden 396,5 g Perfluoroctylbromid zurückgewonnen.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₅Br | CF₃(CF₂)₆Br | CF₃(CF₂)₇Br |
| 0 min | 0,017 | 0,011 | 0,018 | <0,001 | <0,001 | 99,83 |
| 15 min | 0,0065 | 0,0045 | 0,0066 | <0,001 | <0,001 | 99,85 |
| 1 h | <0,0005 | <0,0005 | 0,0006 | <0,001 | <0,001 | 99,86 |
| 8 h | <0,0005 | <0,0005 | 0,0006 | <0,001 | <0,001 | 99,85 |

Das zurückgewonnene Perfluoroctylbromid wird 2 Stunden mit 50 ml 2m-Natronlauge unter intensivem Rühren auf Rückflußtemperatur erhitzt, nach Abtrennen von der Natronlauge mit destilliertem Wasser gewaschen und bei Normaldruck destilliert. Das bei einer Kopftemperatur von 141 °C übergehende Perfluoroctylbromid besitzt eine Reinheit von über 99,9 %.

### Beispiel 2

Beispiel 1 wird wiederholt mit der Abänderung, daß dem Perfluoroctylbromid vor der Bestrahlung 0,3 g Brom zugesetzt werden (und die Behandlung mit Natriumhydrogencarbonatlösung statt Natronlauge erfolgt). Das Molverhältnis von Brom zur Summe der Perfluoralkyliodide beträgt 5 : 1. Die Reaktionstemperatur liegt bei 38 °C. Nach einer Bestrahlungsdauer von 10 Minuten, 20 Minuten, 30 Minuten, 1 Stunde, 2 Stunden und 8 Stunden werden Proben gezogen und mit wäßriger Natriumhydrogencarbonatlösung, die 0,5 g NaHCO₃ je 100 g Lösung enthält, und mit destilliertem Wasser ausgeschüttelt. Die Ergebnisse der kapillargaschromatographischen Analyse sind in der folgenden Tabelle angegeben.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₅Br | CF₃(CF₂)₆Br | CF₃(CF₂)₇Br |
| 0 min | 0,017 | 0,011 | 0,018 | <0,001 | <0,001 | 99,83 |
| 10 min | 0,0077 | 0,0052 | 0,0085 | 0,0035 | 0,0024 | 99,85 |
| 20 min | 0,0025 | 0,0021 | 0,0033 | 0,0058 | 0,0036 | 99,86 |
| 30 min | 0,0008 | 0,0011 | 0,0014 | | 0,0038 | 99,84 |
| 1 h | <0,0005 | <0,0005 | <0,0005 | 0,0083 | 0,0043 | 99,86 |
| 2 h | <0,0005 | <0,0005 | <0,0005 | 0,0090 | 0,0055 | 99,86 |
| 8 h | <0,0005 | <0,0005 | <0,0005 | 0,014 | 0,0098 | 99,85 |

Insgesamt werden 395 g Perfluoroctylbromid zurückisoliert.

### Beispiel 3

Beispiel 1 wird wiederholt mit der Abänderung, daß die Behandlung mit Natronlauge entfällt und daß das durch die Bestrahlung gebildete elementare Iod während der Bestrahlung außerhalb der Bestrahlungsapparatur durch Ausrühren mit verdünnter wäßriger Natriumthiosulfatlösung, die 5 g Na₂S₂O₃ auf 100 g Lösung enthält, entfernt wird und das danach farblose Perfluoroctylbromid nach Abtrennung von der wäßrigen Phase der Bestrahlungsapparatur wieder zugeführt wird. Nach 10 Minuten, 20 Minuten, 30 Minuten und 1 Stunde werden Proben für die kapillargaschromatographische Analyse gezogen.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₅Br | CF₃(CF₂)₆Br | CF₃(CF₂)₇Br |
| 0 min | 0,017 | 0.011 | 0,018 | <0,001 | <0,001 | 99,83 |
| 10 min | 0,0072 | 0,0048 | 0,0071 | <0,001 | <0,001 | 99,85 |
| 20 min | 0,0011 | 0,0013 | 0,0016 | 0,0015 | <0,001 | 99,86 |
| 30 min | <0,0005 | <0,0005 | <0,0005 | 0,0013 | <0,001 | 99,87 |
| 1 h | <0,0005 | <0,0005 | <0,0005 | <0,001 | <0,001 | 99,86 |

Insgesamt werden 391 g Perfluoroctylbromid zurückisoliert.

### Beispiel 4

In die im Beispiel 1 beschriebene Bestrahlungsapparatur werden 350 g Perfluoroctylbromid, das 0,021 % (m/m) Perfluorhexyliodid, 0,013 % (m/m) Perfluorheptyliodid und 0,020 % (m/m) Perfluoroctyliodid enthält, eingefüllt und 5 ml Isopropanol und 45 ml 1 molare wäßrige Natronlauge zugesetzt. Die zweiphasige Mischung wird unter Bestrahlung mit dem Quecksilber-Hochdruckstrahler mit Tauchrohr aus Duran 50 und unter Kühlung intensiv gerührt. Der pH-Wert bleibt während der 4stündigen Bestrahlung alkalisch. Nach 1 Stunde und nach 4 Stunden wird vom Perfluoroctylbromid eine Probe gezogen und das Perfluoroctylbromid dreimal mit destilliertem Wasser extrahiert. Die Ergebnisse der kapillargaschromatographischen Analyse zeigt die nachfolgende Tabelle. Insgesamt werden 342 g Perfluoroctylbromid zurückgewonnen.

| Bestrahlungsdauer | Gehalt [% (m/m)] an: | | | |
|---|---|---|---|---|
| | CF₃(CF₂)₅I | CF₃(CF₂)₆I | CF₃(CF₂)₇I | CF₃(CF₂)₇Br |
| 0 min | 0,021 | 0,013 | 0,020 | 99,81 |
| 1 h | <0,0005 | <0,0005 | 0,0007 | 99,86 |
| 4 h | <0,0005 | <0,0005 | <0,0005 | 99,87 |

### Beispiel 5

In die im Beispiel 1 beschriebene Bestrahlungsapparatur werden 300,5 g Perfluoroctylbromid, das 0,021 % (m/m) Perfluorhexyliodid, 0,013 % (m/m) Perfluorheptyliodid und 0,020 % (m/m) Perfluoroctyliodid enthält, eingefüllt und 100 ml 0,1 molare wäßrige Natronlauge zugesetzt. Durch die zweiphasige Mischung wird ein schwacher Sauerstoffstrom von 1 bar Druck geleitet und intensiv gerührt. Das Perfluoroctylbromid wird unter Kühlung bei einer Innentemperatur von 39 °C mit einem Quecksilber-Hochdruckstrahler mit Tauchrohr aus Duran 50 und unter weiterer Sauerstoffeinleitung bestrahlt. Nach 1 Stunde wird vom Perfluoroctylbromid eine Probe gezogen und nach 8 Stunden das Perfluoroctylbromid von der oberen alkalischen wäßrigen Phase abgetrennt, mit destilliertem Wasser ausgeschüttelt und kapillargaschromatographisch untersucht. Insgesamt werden 298 g Perfluoroctylbromid zurückgewonnen.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₅Br | CF₃(CF₂)₆Br | CF₃(CF₂)₇Br |
| 0 min | 0,021 | 0,013 | 0,020 | <0,001 | <0,001 | 99,81 |
| 1 h | <0,0005 | <0,0005 | <0,0005 | 0,0015 | <0,001 | 99,87 |
| 8 h | <0,0005 | <0,0005 | <0,0005 | <0,001 | <0,001 | 99,86 |

Die alkalische wäßrige Phase enthält nach der Bestrahlung in Spuren die Natriumsalze von Perfluoralkancarbonsäuren, die sich aus den Perfluoralkyliodiden gebildet haben.

### Beispiel 6

In die im Beispiel 1 beschriebene Bestrahlungsapparatur werden 300,5 g Perfluoroctylbromid, das 0,021 % (m/m) Perfluorhexyliodid, 0,013 % (m/m) Perfluorheptyliodid und 0,020 % (m/m) Perfluoroctyliodid enthält, eingefüllt und 100 ml 0,1 molare wäßrige Natronlauge zugesetzt. Das Perfluoroctylbromid und die Natronlauge werden unter Rühren durch mehrmaliges teilweises Evakuieren der Gasphase und Einleiten von Stickstoff in die beiden flüssigen Phasen vom gelösten Luftsauerstoff befreit. Das Perfluoroctylbromid wird unter Kühlung bei einer Innentemperatur von 35 °C mit einem Quecksilber-Hochdruckstrahler mit Tauchrohr aus Duran 50 und unter weiterer schwacher Stickstoffeinleitung und Rühren bestrahlt. Nach 1 Stunde wird vom Perfluoroctylbromid eine Probe gezogen und nach 8 Stunden das Perfluoroctylbromid von der oberen alkalischen wäßrigen Phase abgetrennt, mit destilliertem Wasser ausgeschüttelt und kapillargaschromatographisch untersucht. Insgesamt werden 297 g Perfluoroctylbromid zurückgewonnen.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₁₂CF₃ | CF₃(CF₂)₁₄CF₃ | CF₃(CF₂)₇Br |
| 0 min | 0,021 | 0,013 | 0,020 | <0,001 | <0,001 | 99,81 |
| 1 h | 0,013 | 0,0093 | 0,020 | 0,0012 | <0,001 | 99,83 |
| 8 h | <0,0005 | <0,0005 | 0,011 | 0,0070 | 0,016 | 99,83 |

In Abwesenheit von Sauerstoff dimerisieren die photochemisch gebildeten Perfluoralkylradikale R_{F} zu Perfluoralkanen R_{F}-R_{F}, die sich destillativ von den Perfluoralkylbromiden der Formel R_{F}Br abtrennen lassen.

### Beispiel 7

In eine zylindrische Bestrahlungsapparatur (Innendurchmesser: 5,5 cm, Höhe: 20 cm) mit Mantelkühlung, einer Heraeus-Labortauchlampe mit dem Quecksilberdampf-Niederdruckstrahler TNN 15/32 und einem Tauchrohr aus Quarz (nahezu monochromatischer Strahlungsfluß bei 254 nm: circa 6 Watt), Intensivkühler mit CaCl₂-Trockenrohr, Innenthermometer und Magnetrührfisch werden 400 g Perfluoroctylbromid und 0,3 g Brom eingefüllt. Das Perfluoroctylbromid enthält nach kapillargaschromatographischer Bestimmung 0,017 % (m/m) Perfluorhexyliodid, 0,011 % (m/m) Perfluorheptyliodid und 0,018 % (m/m) Perfluoroctyliodid. Das Molverhältnis von Brom zur Summe der Perfluoralkyliodide beträgt 5 : 1. Unter UV-Bestrahlung wird die Lösung bei 30 bis 35 °C gerührt. Nach einer Bestrahlungsdauer von 1 Stunde und 8 Stunden werden Proben für die kapillargaschromatographische Analyse gezogen und nach 8 Stunden der gesamte Ansatz zweimal mit 2m-Natronlauge und mit destilliertem Wasser ausgeschüttelt. Insgesamt werden 391 g Perfluoroctylbromid zurückgewonnen.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₅Br | CF₃(CF₂)₆Br | CF₃(CF₂)₇Br |
| 0 min | 0,017 | 0,011 | 0,018 | <0,001 | <0,001 | 99,83 |
| 1 h | 0,0097 | 0,0070 | 0,013 | 0,030 | 0,059 | 99,71 |
| 8 h | 0,0065 | <0,001 | 0,0079 | 0,085 | 0,24 | 99,43 |

Den Werten der Tabelle ist zu entnehmen, daß kurzwelliges UV-Licht der Wellenlänge 254 nm in geringem Umfang die Kohlenstoffkette von Perfluoroctylbromid abbaut, wodurch sich die Reinheit des Perfluoroctylbromids verschlechtert.

### Beispiel 8

Beispiel 7 wird wiederholt mit der Abänderung, daß die Bestrahlung ohne Zusatz von 0,3 g Brom bei 18 °C durchgeführt wird. Während der Bestrahlung mit UV-Licht der Wellenlänge von 254 nm wird in Spuren elementares Brom aus dem Perfluoroctylbromid abgespalten, und das Perfluoroctylbromid färbt sich hellbraun. Nach einer Bestrahlungszeit von 8 Stunden und Natronlauge-Behandlung wie im Beispiel 7 beschrieben werden 396 g Perfluoroctylbromid zurückgewonnen.

| Bestrahlungsdauer | Gehalt [% (m/m)] an: | | | | | |
|---|---|---|---|---|---|---|
| | CF₃(CF₂)₅I | CF₃(CF₂)₆I | CF₃(CF₂)₇I | CF₃(CF₂)₅Br | CF₃(CF₂)₆Br | CF₃(CF₂)₇Br |
| 0 min | 0,017 | 0,011 | 0,018 | <0,001 | <0,001 | 99,83 |
| 8 h | 0,0064 | 0,0014 | 0,012 | 0,11 | 0,25 | 99,34 |

## Patentansprüche

1. Verfahren zur Entfernung von Verunreinigungen aus einer Verbindung oder Verbindungen der Formel
X(CF₂)ₙBr (I) ,
in der bedeuten
X = F, (CF₃)₂CF oder Br und
n = eine Zahl von 2 bis 16,
dadurch gekennzeichnet, daß das zu reinigende Gemisch mit einer elektromagnetischen Strahlung im Wellenlängenbereich von 230 bis 500 nm bestrahlt, während oder nach der Bestrahlung mit mindestens einem der folgenden Mittel: Aktivkohle, einem feinteiligen Feststoff aus der Reihe Cu, CuI, Ag, Mg, Zn, Al, Mn, Fe, Co, Ni und einem Alkalimetallborhydrid, Chlor, Brom, Sauerstoff, wäßrige Lösungen von H₂O₂, anderen anorganischen peroxidischen Verbindungen oder Alkalimetallsalzen mit folgenden Anionen: SO₃²⁻, S₂O₃²⁻, PO₃³⁻, NO₂⁻, CO₃²⁻, HCO₃⁻, BrO₃⁻, ClO₃⁻, wäßrige oder alkoholische Lösungen von Alkalimetallhydroxiden, -iodiden, -alkoholaten oder niedrigen aliphatischen Alkoholen in Kontakt gebracht, nach Beendigung der Behandlung das Mittel abgetrennt und das zu reinigende Gemisch, falls erforderlich, mit Wasser gewaschen und destilliert wird.

2. Verfahren nach Anspruch 1, dadurch gekennzeichnet, daß das zu reinigende Gemisch flüssig ist.

3. Verfahren nach Anspruch 1 oder 2, dadurch gekennzeichnet, daß das zu reinigende Gemisch durch eine Zone, in der die Bestrahlung erfolgt, und anschließend durch eine Zone, in der es mit dem in Anspruch 1 genannten Mittel in Kontakt kommt, gegebenenfalls im Kreislauf mehrfach hintereinander, geführt wird.

4. Verfahren nach einem oder mehreren der Ansprüche 1 bis 3, dadurch gekennzeichnet, daß die Bestrahlung bei einer Temperatur erfolgt, die 5 bis 80 °C über dem Schmelzpunkt des zu reinigenden Gemisches liegt, jedoch nicht höher als 200 °C ist.

5. Verfahren nach einem oder mehreren der Ansprüche 1 bis 4, dadurch gekennzeichnet, daß eine elektromagnetische Strahlung verwendet wird, die zu mindestens 50 % ihrer Gesamtintensität im Wellenlängenbereich von 280 bis 450 nm liegt und deren Intensität im Wellenlängenbereich kleiner als 260 nm unter 10 % der Gesamtintensität liegt.

6. Verfahren nach einem oder mehreren der Ansprüche 1 bis 5, dadurch gekennzeichnet, daß das Mittel, mit dem das zu reinigende Gemisch in Kontakt gebracht wird, eine wäßrige Lösung eines Alkalimetallhydroxids, -carbonats, -hydrogencarbonats oder -thiosulfats ist.

7. Verfahren nach einem oder mehreren der Ansprüche 1 bis 5, dadurch gekennzeichnet, daß das Mittel, mit dem das zu reinigende Gemisch in Kontakt gebracht wird, feinteiliges Mg oder Zn oder Aktivkohle ist.

8. Verfahren nach einem oder mehreren der Ansprüche 1 bis 5, dadurch gekennzeichnet, daß mit einer elektromagnetischen Strahlung im Wellenlängenbereich von 350 bis 500 nm bestrahlt wird und das Mittel, mit dem das zu reinigende Gemisch in Kontakt gebracht wird, Chlor oder Brom ist.

9. Verfahren nach einem oder mehreren der Ansprüche 1 bis 8, dadurch gekennzeichnet, daß ein Gemisch eingesetzt wird, das im wesentlichen aus mindestens einer Verbindung der Formel (I) besteht, in der X = F oder Br und n = eine Zahl von 4 bis 10 bedeuten.

## Claims

1. A process for removing impurities from a compound or compounds of the formula
X(CF₂)ₙBr (I)
in which
X is F, (CF₃)₂CF or Br and
n is a number from 2 to 16,
which comprises irradiating the mixture to be purified with electromagnetic radiation in the wavelength range from 230 to 500 nm, bringing the mixture into contact, during or after the irradiation, with at least one of the following agents: active charcoal, a finely divided solid from the series comprising Cu, CuI, Ag, Mg, Zn, Al, Mn, Fe, Co, Ni and an alkali metal borohydride, chlorine, bromine, oxygen, aqueous solutions of H₂O₂, other inorganic peroxidic compounds or alkali metal salts with the following anions: SO₃²⁻, S₂O₃²⁻, PO₃³⁻, NO₂⁻, CO₃²⁻, HCO₃⁻, BrO₃⁻, ClO₃⁻, aqueous or alcoholic solutions of alkali metal hydroxides, iodides or alcoholates or lower aliphatic alcohols, removing the agent after the treatment has ended and, if necessary, washing the mixture to be purified with water and distilling it.

2. The process as claimed in claim 1, wherein the mixture to be purified is liquid.

3. The process as claimed in claim 1 or 2, wherein the mixture to be purified is passed through a zone in which the irradiation takes place and then through a zone in which it comes into contact with the agent mentioned in claim 1, if necessary in circulation several times in succession.

4. The process as claimed in one or more of claims 1 to 3, wherein the irradiation is carried out at a temperature which is 5 to 80°C above the melting point of the mixture to be purified, but is not higher than 200°C.

5. The process as claimed in one or more of claims 1 to 4, wherein electromagnetic radiation is used which lies in the wavelength range from 280 to 450 nm to the extent of at least 50% of its total intensity and in which the intensity in the wavelength range below 260 nm is less than 10% of the total intensity.

6. The process as claimed in one or more of claims 1 to 5, wherein the agent with which the mixture to be purified is brought into contact is an aqueous solution of an alkali metal hydroxide, carbonate, bicarbonate or thiosulfate.

7. The process as claimed in one or more of claims 1 to 5, wherein the agent with which the mixture to be purified is brought into contact is finely divided Mg or Zn or active charcoal.

8. The process as claimed in one or more of claims 1 to 5, wherein the mixture is irradiated with electromagnetic radiation in the wavelength range from 350 to 500 nm and the agent with which the mixture to be purified is brought into contact is chlorine or bromine.

9. The process as claimed in one or more of claims 1 to 8, wherein a mixture which essentially consists of at least one compound of the formula (I) in which X is F or Br and n is a number from 4 to 10 is employed.

## Revendications

1. Procédé pour éliminer des impuretés se trouvant dans un ou plusieurs composés de formule :
X(CF₂)ₙBr (I)
dans laquelle :
X est F, (CF₃)₂CF ou Br, et
n est un nombre de 2 à 16,
caractérisé en ce que le mélange à purifier est exposé à un rayonnement électromagnétique sur l'intervalle de longueurs d'onde de 230 à 500 nm ; pendant ou après l'exposition, il est mis en contact avec au moins l'un des produits suivants : du charbon actif, un matériau solide finement divisé choisi parmi l'ensemble comprenant Cu, CuI, Ag, Mg, Zn, Al, Mn, Fe, Co, Ni et un borohydrure d'un métal alcalin, le chlore, le brome, l'oxygène, les solutions aqueuses de H₂O₂, d'autres composés peroxydiques minéraux ou sels de métaux alcalins avec les anions suivants : SO₃²⁻, S₂O₃²⁻, PO₃³⁻, NO₂⁻, CO₃²⁻, HCO₃⁻, BrO₃⁻, ClO₃⁻, des solutions aqueuses ou alcooliques d'hydroxydes, d'iodures ou d'alcoolates de métaux alcalins, ou d'alcools aliphatiques inférieurs ; après la fin du traitement, on sépare le produit ; et, si nécessaire, on lave le produit à l'eau et on le distille.

2. Procédé selon la revendication 1, caractérisé en ce que le mélange à purifier est liquide.

3. Procédé selon la revendication 1 ou 2, caractérisé en ce que le mélange à purifier traverse une zone dans laquelle a lieu l'irradiation, puis une zone dans laquelle il est mis en contact avec le produit mentionné dans la revendication 1, éventuellement en circuit fermé plusieurs fois de suite.

4. Procédé selon l'une ou plusieurs des revendications 1 à 3, caractérisé en ce que l'irradiation a lieu à une température qui est de 5 à 80°C supérieure au point de fusion du mélange à purifier, mais qui n'est pas supérieure à 200°C.

5. Procédé selon l'une ou plusieurs des revendications 1 à 4, caractérisé en ce qu'on utilise un rayonnement électromagnétique, qui, pour au moins 50 % de son intensité totale, est compris dans l'intervalle de longueurs d'onde de 280 à 450 nm, et dont l'intensité dans l'intervalle de longueurs d'onde inférieur à 260 nm est inférieure à 10 % de l'intensité totale.

6. Procédé selon l'une ou plusieurs des revendications 1 à 5, caractérisé en ce que le produit avec lequel est mis en contact le mélange à purifier est une solution aqueuse d'un hydroxyde, d'un carbonate, d'un hydrogénocarbonate ou d'un thiosulfate d'un métal alcalin.

7. Procédé selon l'une ou plusieurs des revendications 1 à 5, caractérisé en ce que le produit avec lequel est mis en contact le mélange à purifier est constitué de Mg ou de Zn finement divisé, ou de charbon actif.

8. Procédé selon l'une ou plusieurs des revendications 1 à 5, caractérisé en ce qu'on procède à une irradiation avec un rayonnement électromagnétique dans l'intervalle de longueurs d'onde de 350 à 500 nm, et que le produit avec lequel est mis en contact le mélange à purifier est le chlore ou le brome.

9. Procédé selon l'une ou plusieurs des revendications 1 à 8, caractérisé en ce qu'on utilise un mélange qui pour l'essentiel est constitué d'au moins un composé de formule (I) dans laquelle X est F ou Br, et n est un nombre de 4 à 10.
